# EUROPEAN PATENT APPLICATION

(11) **EP 3 575 392 A1**
(43) Date of publication of application: **04.12.2019**
(21) Application number: 18745322.0
(22) Date of filing: 25.01.2018
(51) Int. Cl.: C12N 5/077, C12N 5/0735, C12N 5/0775, C12N 5/074, C12N 5/10

(54) **MEDIUM FOR INDUCING DIFFERENTIATION OF STEM CELLS INTO MESODERMAL CELLS AND METHOD FOR PRODUCING MESODERMAL CELLS**

(30) Priority: 26.01.2017 JP 2017012333; 24.02.2017 JP 2017033322
(71) Applicant: Osaka University, Suita-shi, Osaka 565-0871 (JP); Kyowa Hakko Bio Co., Ltd., Tokyo 100-8185 (JP)
(72) Inventor: AKASHI, Mitsuru, Suita-shi Osaka 565-0871 (JP); FUKUMOTO, Ken, Tokyo 100-8185 (JP); SHOJI, Shinichiro, Tokyo 100-8185 (JP)
(74) Representative: Hoffmann Eitle
(86) International application number: PCT/JP2018/002321
(87) International publication number: WO 2018/139548

(57) **Abstract**

The present invention provides a medium for inducing the differentiation of stem cells into mesodermal cells, the medium comprising a ROCK inhibitor, a bone morphogenetic protein (BMP), a fibroblast growth factor (FGF), and activin. The present invention also provides a method for producing mesodermal cells from stem cells using the medium, and a method for producing cardiac progenitor cells or myocardiocytes from stem cells using the medium. The present invention furthermore provides a composition for assisting the induction of differentiation of stem cells into mesodermal cells, the composition comprising a ROCK inhibitor, a BMP, an FGF, and activin. The present invention additionally provides cell groups obtained by the above methods.

## Description

### Technical Field

The present disclosure relates to a medium for inducing the differentiation of stem cells into mesodermal cells, and to a method for producing mesodermal cells from stem cells using the medium.

### Background Art

According to prior art, for example, in order to obtain desired cells through differentiation induction from stem cells by suspension culture, as a preliminary stage, the formation of embryoid bodies (EB) is required. After such a preliminary-stage operation, differentiation induction according to various cells is performed to make stem cells differentiate into target cells (Non-Patent Literatures 1 to 7). Yamanaka et al., have developed a method in which EBs are once formed, and the obtained EBs are dissociated and reaggregated, thereby efficiently producing cardiomyocytes from stem cells (Patent Literature 1).

### Citation List

### Patent Literatures

Patent Literature 1: WO 2014/185358

### Non-Patent Literatures

Non-Patent Literature 1: Izhak Kehat et al., J Clin Invest. 2001 Aug; 108 (3): 407-14
Non-Patent Literature 2: Christine Mummery et al., Circulation. 2003 Jun 3; 107 (21): 2733-40
Non-Patent Literature 3: Byung Sun Yoon et al., Differentiation. 2006 Apr; 74 (4): 149-59
Non-Patent Literature 4: Michael A Laflamme et al., Nat Biotechnol. 2007 Sep; 25 (9): 1015-24
Non-Patent Literature 5: Katsuhisa Matsumura et al., Biochem Biophys Res Commun. 2015 Jun 19; 462 (1): 52-7
Non-Patent Literature 6: Henning Kemph et al., Stem Cell Reports. 2014 Dec 9; 3 (6): 1132-46
Non-Patent Literature 7: Shogo Tohyama et al., Cell Metab. 2016 Apr 12; 23 (4): 663-74

### Summary of Invention

### Technical Problem

When stem cells are induced to differentiate into target cells, it is desirable that the differentiation into target cells takes place as efficiently as possible. In addition, in the case where stem cells are induced to differentiate into target cells by suspension culture, a conventional method has problems in that the optimal EB formation period has to be previously examined, and the EB formation takes time. Therefore, a quick, simple, and high-efficiency method for inducing the differentiation of stem cells is required.

### Solution to Problem

The present inventors have conducted extensive research. As a result, they have found that when cells are cultured in a medium comprising a Rho-associated kinase (Rho-associated coiled-coil forming kinase: ROCK) inhibitor, a bone morphogenetic protein (BMP), a fibroblast growth factor (FGF), and an activin, stem cells can be induced to differentiate into mesodermal cells with high efficiency, and thus accomplished the present invention.

That is, the present invention provides:
[1] A medium for inducing the differentiation of stem cells into mesodermal cells, comprising a ROCK inhibitor, a bone morphogenetic protein (BMP), a fibroblast growth factor (FGF), and an activin;
[2] The medium according to [1], wherein the ROCK inhibitor is Y-27632;
[3] The medium according to [1] or 2, wherein the BMP is BMP4;
[4] The medium according to any one of [1] to [3], wherein the FGF is FGF-2;
[5] The medium according to any one of [1] to [4], wherein the activin is activin A;
[6] The medium according to any one of [1] to [5], wherein the mesodermal cells are cardiac progenitor cells or cardiomyocytes;
[7] The medium according to any one of [1] to [6], wherein the stem cells are pluripotent stem cells or mesenchymal stem cells;
[8] A method for producing mesodermal cells from stem cells, comprising a step of culturing stem cells in the medium according to any one of [1] to [7];
[9] A method for producing cardiac progenitor cells or cardiomyocytes from stem cells, comprising:
   a step (1) of culturing stem cells in the medium according to any one of [1] to [7], and then;
   a step (2) of culturing in a medium comprising a Wnt inhibitor;
[10] The method according to [9], wherein the Wnt inhibitor is IWR-1 and IWP-2;
[11] The method according to any one of [8] to [10], wherein the culture is suspension culture;
[12] The method according to any one of [8] to [11], wherein the stem cells are pluripotent stem cells or mesenchymal stem cells;
[13] A composition for assisting the induction of the differentiation of stem cells into mesodermal cells, comprising a ROCK inhibitor, a BMP, an FGF, and an activin; and
[14] A cell group obtainable by the method according to any one of [8] to [12], comprising 90% or more mesodermal cells.

### Advantageous Effects of Invention

According to the present invention, stem cells can be induced to differentiate into mesodermal cells with high efficiency. Further, in the case of using suspension culture, a large number of stem cells can be induced to differentiate into mesodermal cells in a quick and simple manner without through the EB formation period.

### Brief Description of Drawings

[Fig. 1] Fig. 1 shows the culture conditions of Example 1 (Conditions 1 to 4).
[Fig. 2] Fig. 2 shows changes in the viable cell density under Conditions 1 to 4.
[Fig. 3] Fig. 3 shows the proportion of cTnT positive cells under Conditions 1 to 4.
[Fig. 4] Fig. 4 shows the culture conditions of Example 2 (Conditions A to F).
[Fig. 5] Fig. 5 shows changes in the viable cell density under Conditions A to F.
[Fig. 6] Fig. 6 shows the rate of cTnT-positive cells under Conditions A, D, and E.

### Description of Embodiments

The present invention provides a medium for inducing differentiation from stem cells into mesodermal cells, wherein the medium is for producing mesodermal cells from stem cells and comprises a ROCK inhibitor, a BMP, an activin, and an FGF (hereinafter sometimes referred to as "medium of the present invention"); a method for culturing stem cells in the medium to produce mesodermal cells (hereinafter sometimes referred to as "method of the present invention"); a composition for assisting the induction of the differentiation of stem cells into mesodermal cells (hereinafter sometimes referred to as "composition of the present invention"); and a cell group obtainable by the method of the present invention (hereinafter sometimes referred to as "cell group of the present invention").

### 1. Medium of the Present Invention

The medium of the present invention is a medium for inducing the differentiation of stem cells into mesodermal cells, comprising a ROCK inhibitor, a BMP, an FGF, and an activin. The medium of the present invention is a medium obtained by adding components comprising a ROCK inhibitor, a BMP, an FGF, and an activin to a basal medium for stem cells.

As used herein, "stem cells" refers to immature cells having self-replication ability and differentiation/proliferation ability. A hierarchy exists in stem cells. It is known that upper-level, undifferentiated stem cells have higher self-replication ability and also have higher pluripotency, that is, such cells can differentiate into various cell lines; meanwhile, lower-level stem cells have lower self-replication ability, and they can only differentiate into specific cell lines.

With respect to stem cells to which the medium of the present invention can be applied, the species from which the cells are derived are not particularly limited. Examples thereof include rodents such as rats, mice, hamsters, and guinea pigs, lagomorpha such as rabbits, ungulate such as pigs, cows, goats, and sheep, carnivora such as dogs and cats, and primates such as humans, apes, rhesus monkeys, marmosets, orangutans, and chimpanzees.

Stem cells include, depending on the differentiation ability, pluripotent stem cells, multipotent stem cells, unipotent stem cells, and the like.

As used herein, "pluripotent stem cells" means stem cells that can differentiate into any cells that may be present in a living body (i.e., having pluripotency) and have proliferation ability. Examples of pluripotent stem cells include, but are not limited to, induced pluripotent stem cells (iPS cells), embryonic stem cells (ES cells), embryonic stem cells derived from cloned embryos obtained by nuclear transplantation (ntES cells), spermatogonial stem cells (GS cells), embryonic germ cells (EG cells), and pluripotent cells derived from cultured fibroblasts or bone marrow stem cells (Muse cells). The pluripotent stem cells used may be produced by a known method, or may also be a commonly available cell strain. Examples of ES cells include, but are not limited to, KhES1 and KhES3.

According to one embodiment, the pluripotent stem cells used in the present invention are iPS cells. iPS cells are somatic cell-derived artificial stem cells having pluripotency and proliferation ability, which are produced by introducing a specific reprogramming factor in the form of a nucleic acid or a protein into somatic cells.

Examples of genes included in reprogramming factors include Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall1, Sall4, Esrrb, Nr5a2, Tbx3, and Glis1. These reprogramming factors may be used alone or in combination.

Examples of combinations of reprogramming factors include the combinations described in WO 2007/069666, WO 2008/118820, WO 2009/007852, WO 2009/032194, WO 2009/058413, WO 2009/057831, WO 2009/075119, WO 2009/079007, WO 2009/091659, WO 2009/101084, WO 2009/101407, WO 2009/102983, WO 2009/114949, WO 2009/117439, WO 2009/126250, WO 2009/126251, WO 2009/126655, WO 2009/157593, WO 2010/009015, WO 2010/033906, WO 2010/033920, WO 2010/042800, WO 2010/050626, WO 2010/056831, WO 2010/068955, WO 2010/098419, WO 2010/102267, WO 2010/111409, WO 2010/111422, WO 2010/115050, WO 2010/124290, WO 2010/147395, WO 2010/147612, Huangfu D, et al., (2008), Nat. Biotechnol., 26: 795-797, Shi Y, et al. (2008), Cell Stem Cell, 2: 525-528, Eminli S, et al., (2008), Stem Cells. 26: 2467-2474, Huangfu D, et al., (2008), Nat Biotechnol. 26: 1269-1275, Shi Y, et al., (2008), Cell Stem Cell, 3, 568-574, Zhao Y, et al., (2008), Cell Stem Cell, 3: 475-479, Marson A (2008), Cell Stem Cell, 3, 132-135, Feng B, et al., (2009), Nat Cell Biol. 11: 197-203, R.L. Judson et al., (2009), Nat. Biotech., 27: 459-461, Lyssiotis CA, et al., (2009), Proc Natl Acad Sci U SA. 106: 8912-8917, Kim JB, et al., (2009), Nature. 461: 649-643, Ichida JK, et al., (2009), Cell Stem Cell. 5: 491-503, Heng JC, et al., (2010), Cell Stem Cell. 6: 167-74, Han J, et al., (2010), Nature. 463: 1096-100, Mali P, et al., (2010), Stem Cells. 28: 713-720, Maekawa M, et al., (2011), Nature. 474: 225-9, and the like.

The iPS cells used in the present invention may be produced by a known method, or may also be a commonly available cell strain. Examples of strains of human-derived iPS cells include, but are not limited to, 253G1 (RIKEN Cell Bank No. HPS0002), 201B7 (RIKEN Cell Bank No. HPS0063), 409B2 (RIKEN Cell Bank No. HPS0076), 454E2 (RIKEN Cell Bank No. HPS0077), 606A1 (RIKEN Cell Bank No. HPS0328), 610B1 (RIKEN Cell Bank No. HPS0331), 648A1 (RIKEN Cell Bank No. HPS0360), MYH (Patent Literature 1), 427F1 (Patent Literature 1), 457C1 (Patent Literature 1), 604A1 (Patent Literature 1), HiPS-RIKEN-1A (RIKEN Cell Bank No. HPS0003), HiPS-RIKEN-2A (RIKEN Cell Bank No. HPS0009), HiPS-RIKEN-12A (RIKEN Cell Bank No. HPS0029), and Nips-B-2 (RIKEN Cell Bank No. HPS0223). Examples of strains of iPS cells derived from non-human animals include, but are not limited to, iPS-MEF-Ng-20D-17, iPS-MEF-Ng-178B-5, iPS-MEF-Fb/Ng-440A-3, iPS-MEF-Ng-492B-4, iPS-Stm-FB/gfp-99-1, iPS-Stm-FB/gfp-99-3, iPS-Hep-FB/Ng/gfp-103C-1, and iPS-L1, iPS-S1. Alternatively, it is also possible to use disease-specific iPS cells. Examples of such diseases include, but are not limited to, blood system disorders, immune system disorders, endocrine system disorders, metabolic system disorders, visual system disorders, circulatory system disorders, respiratory system disorders, skin/connective tissue disorders, bone/articular system disorders, kidney/urinary system disorders, and syndromes accompanied by chromosomal or genetic changes (see http://cell.brc.riken.jp/ja/hps/hps_diseaselist_index).
The cell strains described above are available from RIKEN BioResource Center (see http://cell.brc.riken.jp/en/) or JCRB Cell Bank (http://cellbank.nibiohn.go.jp/english/).

The iPS cells used in the present invention may be obtained by culturing on feeder cells. Alternatively, the cells may also be obtained by culturing under feeder-free conditions. These culture methods are well known to those skilled in the art.

Preparation methods, culture methods, preservation methods, and the like for the pluripotent stem cells described above are well known to those skilled in the art. For example, the methods described in WO 2014/185358 (Patent Literature 1) (which is incorporated herein by reference) can be used.

Examples of the "multipotent stem cells" described above include somatic stem cells such as mesenchymal stem cells, hematopoietic stem cells, neural stem cells, bone marrow stem cells, and germ stem cells. The multipotent stem cells are preferably mesenchymal stem cells.

Mesenchymal stem cells are undifferentiated cells (somatic stem cells) present in the bone marrow, fat tissue, placental tissue, umbilical cord blood, tooth pulp, and the like of an adult, and the term broadly means a group of stem cells having proliferation ability and pluripotent ability (in particular, differentiation ability into osteocytes, cartilage cells, muscle cells, tendon cells, adipocytes, etc.) and progenitor cells thereof. Examples of mesenchymal stem cells include mesenchymal stem cells derived from human bone marrow (hMSC-BM, manufactured by TaKaRa), mesenchymal stem cells derived from human umbilical cord matrix (hMSC-UC, manufactured by TaKaRa), and mesenchymal stem cells derived from human fat tissue (hMSC-AT, manufactured by TaKaRa).

In the present invention, any stem cells can be suitably used. However, it is preferable to use pluripotent stem cells or mesenchymal stem cells, and it is more preferable to use pluripotent stem cells. The pluripotent stem cells are preferably ES cells or iPS cells, more preferably iPS cells, and particularly preferably human iPS cells.

As used herein, "mesodermal cells" refers to cells that constitute mesoderm-derived tissues. Examples of mesoderm-derived tissues include, but are not limited to, bone, cartilage, spleen, bone marrow, tooth dentin, peritoneal epithelium, kidney, urinary tract, pleural epithelium, adrenal cortex, muscle (except for the sphincter pupillae and musculus dilator pupillae), ovary, uterus, testis, and connective tissue. In addition, examples of mesodermal cells also include, but are not limited to, microglia cells, cardiac progenitor cells, cardiomyocytes, vascular endothelial progenitor cells, vascular endothelial cells, blood cells, and mesenchymal stem cells.

The "basal medium for stem cells" descrived above is not particularly limited as long as it is a medium capable of inducing stem cells to differentiate into mesodermal cells when a ROCK inhibitor, a BMP, an FGF, and an activin are added. The basal medium for stem cells preferably comprises one or more saccharides, one or more inorganic salts, one or more amino acids, one or more vitamins, and one or more minor components. In addition, the basal medium may also suitably comprise an antibiotic, such as kanamycin, for use in a drug sensitivity test.

Examples of the saccharides include monosaccharides, such as glucose, lactose, mannose, fructose, and galactose, and disaccharides, such as sucrose, maltose, and lactose. Among them, glucose is particularly preferable. One saccharide or a combination of two or more saccharides may be added.

Examples of the inorganic salts include calcium chloride, calcium nitrate, copper sulfate pentahydrate, iron(III) nitrate nonahydrate, iron(II) sulfate heptahydrate, magnesium chloride hexahydrate, magnesium sulfate, potassium chloride, sodium chloride, disodium hydrogenphosphate, disodium hydrogenphosphate dihydrate, sodium dihydrogenphosphate, sodium dihydrogenphosphate dihydrate, and zinc sulfate heptahydrate. Any inorganic salt or a combination thereof can be used as long as it is a component that advantageously acts on the induction of the differentiation of stem cells into mesodermal cells.

Examples of the amino acids include alanine, arginine, asparagine, aspartic acid, cystine, cysteine, glutamine, glycine, histidine, glutamic acid, hydroxyproline, isoleucine, leucine, lysine, methionine, phenylalanine, proline, serine, threonine, tryptophan, tyrosine, and valine, and L-form amino acid is preferable. The amino acids may include derivatives, such as derivatives, salts, and hydrates thereof.

Examples of derivatives of arginine include L-arginine hydrochloride and L-arginine monohydrochloride. Examples of derivatives of aspartic acid include sodium L-aspartate monohydrate, L-aspartic acid monohydrate, potassium L-aspartate, and magnesium L-aspartate. Examples of derivatives of cysteine include L-cysteine dihydrochloride and L-cysteine hydrochloride monohydrate. Examples of derivatives of lysine include L-lysine monohydrochloride. Examples of derivatives of glutamic acid include monosodium L-glutamate. Examples of derivatives of asparagine include L-asparagine monohydrate. Examples of derivatives of tyrosine include L-tyrosine disodium dihydrate. Examples of derivatives of histidine include histidine hydrochloride and histidine hydrochloride monohydrate. Examples of derivatives of lysine include L-lysine monohydrochloride.

Examples of the vitamins include ascorbic acid, biotin, choline, folic acid, inositol, niacin, pantothenic acid, pyridoxine, riboflavin, thiamine, vitamin B12, and para-aminobenzoic acid (PABA). Among them, ascorbic acid is preferably added. The vitamins may include derivatives, such as derivatives, salts, and hydrates thereof.

Examples of derivatives of ascorbic acid include ascorbic acid 2-phosphate, ascorbic acid magnesium phosphate, ascorbic acid sodium sulfate, aminopropyl ascorbyl phosphate, and ascorbic acid sodium phosphate. Examples of derivatives of choline include choline chloride. Examples of derivatives of niacin include nicotinic acid, nicotinamide, and nicotinyl alcohol. Examples of derivatives of pantothenic acid include calcium pantothenate, sodium pantothenate, and panthenol. Examples of derivatives of pyridoxine include pyridoxine hydrochloride, pyridoxalisol hydrochloride, pyridoxal phosphate, and pyridoxamine. Examples of derivatives of thiamine include thiamine hydrochloride, thiamine nitrate, bisthiamine nitrate, thiamine dicetyl sulfate, fursultiamine hydrochloride, octotiamine, and benfotiamine.

It is preferable that the minor components are components that advantageously act on the induction of the differentiation of stem cells into mesodermal cells. Examples of the minor components include components that are usually used as medium components, such as glutathione, hypoxanthine, lipoic acid, linolenic acid, phenol red, putrescine, pyruvic acid, thymidine, and NaHCO₃. The minor components may include derivatives, such as derivatives, salts, and hydrates thereof. Examples of derivatives include putrescine dihydrochloride.

As the basal medium for stem cells, a basal medium known to those skilled in the art can be used. Examples thereof include commercially available culture media such as StemPro®-34 (Thermo Fisher Scientific) and mESF Basal Medium (Wako Pure Chemical Industries, Ltd.), as well as MEM (Minimum Essential Medium), BME (Basal Medium Eagle), DMEM (Dulbecco's Modified Eagle Medium), EMEM (Eagle's minimal essential medium), IMDM (Iscove's Modified Dulbecco's Medium), GMEM (Glas-gow's MEM), F12 (Ham's F12 Medium), DMEM/F12 (1:1 mixed medium of DMEM and F12 media), RPMI1640, RD, BMOC-3 (Brinster's BMOC-3 Medium), CMRL-1066, L-15 medium (Leibovitz's L-15 medium), McCoy's 5A, Media 199, MEM αMedia, MCDB105, MCDB131, MCDB153, MCDB201, Williams' medium E, ESF, and the like.

As necessary, it is possible to add serum replacements to the basal medium for stem cells, such as KnockOut™ Serum Replacement (KSR) (Thermo Fisher Scientific), StemSure Serum Replacement (SSR) (Wako Pure Chemical Industries, Ltd.), and PluriQ™ Serum Replacement (Cosmo Bio), or non-serum supplements such as B27 Replacement (Thermo Fisher Scientific).

Examples of such media include media obtained by adding KSR to MEM, BME, DMEM, EMEM, IMDM, DMEM/F12, and RPMI1640, media obtained by adding B27 Replacement to MEM, BME, DMEM, EMEM, IMDM, DMEM/F12, and RPMI1640, and StemPro®-34, preferably media obtained by adding B27 Replacement to MEM, BME, DMEM, EMEM, IMDM, DMEM/F12, and RPMI1640 and StemPro®-34, more preferably media obtained by adding B27 Replacement to RPMI1640 and StemPro®-34, and most preferably StemPro®-34.

The basal medium for stem cells may further comprise an additive commonly used in cell culture. Examples of such additives include, but are not limited to, trisodium L-ascorbyl 2-phosphate, L-glutamine, 1-thioglycerol, and the like.

The kind and amount of the medium to be used can be suitably selected by those skilled in the art according to the kind of cells to be cultured.

Examples of ROCK inhibitors used in the present invention include, but are not limited to, (R)-(+)-trans-N-(4-pyridyl)-4-(1-aminoethyl)-cyclohexanecarboxamide·2HCl·H₂O (Y-27632), 1-(5-isoquinolinesulfonyl)piperazine hydrochloride (HA100), 1-(5-isoquinolinesulfonyl)homopiperazine dihydrochloride (Fasudil/HA-1077), (S)-(+)-2-methyl-4-glycyl-1-(4-methylisoquinolinyl-5-sulfonyl)homopiperidine dihydrochloride (H-1152), 1-(5-isoquinolinesulfonyl)-2-methylpiperazine (H-7), 1-(5-isoquinolinesulfonyl)-3-methylpiperazine (iso-H-7), N-2-(methylamino)ethyl-5-isoquinolinesulfonamide dihydrochloride (H-8), N-(2-aminoethyl)-5-isoquinolinesulfonamide dihydrochloride (H-9), N-[2-(p-bromocinnamylamino)ethyl]-5-isoquinolinesulfonamide dihydrochloride (H-89), N-(2-guanidinoethyl)-5-isoquinolinesulfonamide hydrochloride (HA-1004), N-[(LS)-2-hydroxy-1-phenylethyl]-N-[4-(4-pyridinyl)phenyl]-urea (AS1892802), N-[3-[[2-(4-amino-1,2,5-oxadiazol-3-yl)-1-ethyl-1H-imidazo[4,5-c]pyridin-6-yl]oxy]phenyl]-4-[2-(4-morpholinyl)ethoxy]benzamide (GSK269962), N-(6-fluoro-1H-indazol-5-yl)-2-methyl-6-oxo-4-(4-(trifluoromethyl)phenyl)-1,4,5,6-tetrahydropyridine-3-carboxamide (GSK429286), hydroxyfasudil (HA1100), 2-fluoro-N-[[4-(1H-pyrrolo[2,3-b]pyridin-4-yl)phenyl]methyl]benzene methanamine (OXA06), N-[(3-hydroxyphenyl)methyl]-N'-[4-(4-pyridinyl)-2-thiazolyl]urea (RKI1447), 4-(7-{[(3S)-3-amino-1-pyrrolidinyl]carbonyl}-1-ethyl-1H imidazole[4,5-c]pyridin-2-yl)-1,2,5-oxadiazol-3-amine (SB772077B), N-[2-[2-(dimethylamino)ethoxy]-4-(1H-pyrazol-4-yl)phenyl-2,3-dihydro-1,4-benzodioxine-2-carboxyamide dihydrochloride (SR3677), and 6-chloro-N4-[3,5-difluoro-4-[(3-methyl-1H-pyrrolo[2,3-b]pyridin-4-yl)oxy]phenyl]-2,4-pyrimidine diamine (TC-S7001). In an exemplary embodiment, the ROCK inhibitor used is Y-27632.

The concentration of the ROCK inhibitor used in the present invention is about 0.2 to about 100 µM, for example, about 1 to about 75 µM, about 2 to about 50 µM, or about 5 to about 20 µM. In an exemplary embodiment, the concentration of the ROCK inhibitor used is about 10 µM.

Examples of BMPs used in the present invention include, but are not limited to, BMP2, BMP4, BMP6, and BMP8. In an exemplary embodiment, the BMP used is BMP4. The concentration of the BMP used in the present invention is about 0.5 to about 500 ng/mL, for example, about 1 to about 100 ng/mL, about 2 to about 50 ng/mL, or about 5 to about 20 ng/mL. In an exemplary embodiment, the concentration of the BMP used is about 10 ng/mL.

Examples of FGFs used in the present invention include, but are not limited to, FGF-1, FGF-2, FGF-3, FGF-4, FGF-5, FGF-6, FGF-7, FGF-8, and FGFv9. In an exemplary embodiment, the FGF used is FGF-2. The concentration of the FGF used in the present invention is about 0.1 to about 250 ng/mL, for example, about 0.5 to about 50 ng/mL, about 1 to about 25 ng/mL, or about 2.5 to about 10 ng/mL. In an exemplary embodiment, the concentration of the FGF used is about 5 ng/mL.

Examples of activins used in the present invention include, but are not limited to, activin A, activin B, and activin AB. In an exemplary embodiment, the activin used is activin A. The concentration of the activin used in the present invention is about 0.12 to about 300 ng/mL, for example, about 0.6 to about 60 ng/mL, about 1.2 to about 30 ng/mL, or about 3 to about 12 ng/mL. In an exemplary embodiment, the concentration of the activin used is about 6 ng/mL.

### 2. Method of the Present Invention

The method of the present invention is a method for producing mesodermal cells from stem cells, comprising a step of culturing stem cells in the medium of the present invention described in 1 above.

The culture conditions in the method of the present invention, such as the culture temperature and the culture time, can be suitably selected by those skilled in the art according to the kind of cells to be cultured. For example, in the case of using pluripotent stem cells cultured on feeder cells, the pluripotent stem cells are cultured in the medium of the present invention at 34 to 40°C / 2 to 8% CO₂ for 12 hours to 7 days, and preferably at 35 to 39°C / 3 to 7% CO₂ for 1 to 4 days. Cells are more preferably cultured in the medium of the present invention comprising the above four factors (ROCK inhibitor, BMP, FGF, and activin) at 36 to 38°C / 4 to 6% CO₂ for 6 hours to 42 hours, preferably for 12 hours to 36 hours, and more preferably for 18 hours to 30 hours culture, and then cultured in a medium comprising three factors excluding a ROCK inhibitor (BMP, FGF, and activin) at 36 to 38°C / 4 to 6% CO₂ for 12 hours to 4 days, preferably for 1 to 3 days, more preferably for 36 to 60 hours, and most preferably for 42 to 54 hours. In the case of using pluripotent stem cells cultured under feeder-free conditions, the pluripotent stem cells are cultured in the medium of the present invention at 34 to 40°C / 2 to 8% CO₂ for 12 hours to 7 days, and preferably at 35 to 39°C / 3 to 7% CO₂ for 1 to 4 days, more preferably for 2 to 4 days. The kinds and concentrations of the ROCK inhibitor, BMP, FGF, and activin comprised in the medium are as described in 1 above. In an exemplary embodiment, the ROCK inhibitor, BMP, FGF, and activin used are Y-27632, BMP4, FGF-2, and activin A, respectively.

As a mode of culture in the method of the present invention, adherent culture or suspension culture can be used, and, as a preferred mode, suspension culture can be used. Suspension culture can be performed using a means, method, or device that can maintain cells in a floating state. For example, culture can be performed using an incubator equipped with an impeller blade, such as a single-use bioreactor (Biott Corporation), a single-use bioreactor (Thermo Fischer), a single-use bioreactor (Sartorius Stedim), or a single-use bioreactor (GE Healthcare Life Science). The kind of the incubator to be used and its agitation rate can be suitably selected by those skilled in the art according to the kind of cells to be cultured. Examples of agitation rates include, but are not limited to, 0 to 100 rpm, 20 to 80 rpm, and 45 to 65 rpm.

Mesodermal cells are as described in 1 above. When stem cells are induced to differentiate into mesodermal cells, after culturing stem cells in the medium of the present invention, in order to induce differentiation from mesoderm into a mesoderm-derived tissue, a method well known to those skilled in the art method can be used. For example, by culturing cells in a differentiation medium suitable for differentiation into a desired mesoderm-derived tissue, the cells can be induced to differentiate into desired mesodermal cells (Nathan J Palpant et al., Nature Protocols 2016 Dec; 12(1): 15-31, Cynthia A. Batchelder et al., 2009 Jul; 78 (1): 45-56, etc.).

Alternatively, it is also possible that stem cells are induced to differentiate into mesenchymal stem cells, and the obtained mesenchymal stem cell are differentiated into mesodermal cells. Methods for inducing mesenchymal stem cells to differentiate into mesodermal cells are well known to those skilled in the art. For example, by culturing mesenchymal stem cells in a differentiation medium suitable for differentiation into desired cells, the mesenchymal stem cells can be induced to differentiate into mesodermal cells (Nishiyama et al., 2007 Aug; 25 (8): 2017-2024, etc.)

In another embodiment, the present invention provides a method for producing cardiac progenitor cells or cardiomyocytes from stem cells. The method comprises a step (1) of culturing stem cells in the medium of the present invention described above, and then a step (2) of culturing in a medium comprising a Wnt inhibitor. The step (2) may be followed by a step (3) of culturing in a medium comprising a vascular endothelial cell growth factor (VEGF) and an FGF.

In one embodiment, the above step (2) is a step of culturing in a medium comprising IWR-1 and IWP-2, and the above step (3) is a step of culturing in a medium comprising a VEGF and FGF-2. A Wnt inhibitor refers to a substance that inhibits the Wnt signaling pathway. Examples of Wnt inhibitors include, but are not limited to, IWR-1 (4-[(3aR,4S,7R,7aS)-1,3,3a,4,7,7a-hexahydro-1,3-dioxo-4,7-methano-2H-isoindol-2-yl]-N-8-quinolinyl-benzamide), IWP-2 (N-(6-Methyl-2-benzothiazolyl)-2-[(3,4,6,7-tetrahydro-4-oxo-3-phenylthieno[3,2-d]pyrimidin-2-yl)thio]-acetamide), WntC59 (4-(2-methyl-4-pyridinyl)-N-(4-(3-pyridinyl)phenyl)-benzeneacetamide), IWP4 (N-(6-methyl-2-benzothiazolyl)-2-[[3,4,6,7-tetrahydro-3-(2-methoxyphenyl)-4-oxothieno[3,2-d]pyrimidin-2-yl]thio]-acetamide), and KY0211.

In the case where IWR-1 is used as a Wnt inhibitor, the concentration of IWR-1 used is about 0.4 to about 40 µM, about 0.8 to about 20 µM, or about 2 to about 8 µM, for example. In an exemplary embodiment, the concentration of IWR-1 used is about 4 µM. In the case where IWP-2 is used as a Wnt inhibitor, the concentration of IWP-2 used is about 1 to about 100 µM, about 2 to about 50 µM, or about 5 to about 20 µM, for example. In an exemplary embodiment, the concentration of IWP-2 used is about 10 µM.

The concentration of the VEGF used is about 0.1 to about 100 ng/mL, for example, about 0.5 to about 50 ng/mL, about 1 to about 25 ng/mL, or about 2.5 to about 10 ng/mL. In an exemplary embodiment, the concentration of the VEGF used is about 5 ng/mL. In the case where FGF-2 is used as an FGF, the concentration of FGF-2 used is about 0.1 to about 250 ng/mL, for example, about 1 to about 100 ng/mL, about 2 to about 50 ng/mL, or about 5 to about 20 ng/mL. As specific examples of FGFs, the FGFs described in 1 above can be mentioned. In an exemplary embodiment, FGF-2 is used as an FGF, and the concentration of FGF-2 used is about 10 ng/mL.

The culture conditions, such as the culture temperature and the culture time, can be suitably selected by those skilled in the art according to the kind of desired cells. For example, the step (2) may be a step of culturing at 35 to 39°C / 3 to 7% CO₂ for 1 to 7 days. In addition, the step (3) may be a step of culturing at 35 to 42°C / 3 to 7% CO₂ for 1 to 20 days. More preferably, the step (2) is a step of culturing at 36 to 38°C / 4 to 6% CO₂ for 2 to 4 days, and the step (3) is a step of culturing at 36 to 40°C / 4 to 6% CO₂ for 7 to 15 days.

### 3. Composition of the Present Invention

The composition of the present invention is a composition for assisting the induction of the differentiation of stem cells into mesodermal cells, comprising a ROCK inhibitor, a BMP, an activin, and an FGF. The kinds and concentrations of the ROCK inhibitor, BMP, activin, and FGF comprised in the composition are as described in 1 above. In an exemplary embodiment, the ROCK inhibitor, BMP, activin, and FGF used are Y-27632, BMP4, activin A, and FGF-2, respectively. The composition may be a liquid composition, or may also be a powder composition such as a lyophilized product.

### 4. Cell Group of the Present Invention

The cell group of the present invention is a cell group obtained by the method of the present invention described above, and the cell group comprises about 90% or more mesodermal cells obtained by differentiation induction. In the cell group, the proportion of mesodermal cells is about 90% or more, for example, about 91%, about 92%, about 93%, about 94%, about 95%, about 96%, about 97%, about 98%, about 99%, or more.

It should be appreciated that unless particularly noted, the terms used herein are given their ordinary meanings in the art. Therefore, unless otherwise defined, all the terminologies and scientific and technical terms used herein have the same meanings as generally understood by those skilled in the technical field to which the present invention pertains. The term "about" will be understood by those skilled in the art, which somewhat changes according to the context in which the term is used. "About" typically means numerical values within a range of ±10%, more typically ±5%, more typically ±4%, more typically ±3%, more typically ±2%, and still more typically ±1% of the numerical value prefaced by the term.

Hereinafter, the present invention will be specifically described in further detail through examples. However, the examples do not limit the scope of the present invention. Examples

### Stem Cells and Culture Method

As stem cells, an iPS cell strain 253G1 (pluripotent stem cells) was used (Nakagawa, M. et al., Nature Biotechnology 26: 101-106 (2008)). MEF cells (CF-1 MEF) were used as feeder cells. Maintenance culture was performed in a medium for iPS cells. As the medium for iPS cells, a medium obtained by adding KnockOut™ Serum Replacement (KSR) (final concentration: 20%), 0.1 mM MEM nonessential amino acid solution, 1 mM L-glutamine, 0.1 mM β-mercaptoethanol, and 4 ng/mL FGF-2 to KnockOut™ DMEM/F12 was used. The cultured iPS cells were separated with 1 mg/ml dispase and a cell scraper, and then subcultured (Split Ratio = 1 : 5 to 1 : 6).

### Counting of Viable Cells

Cells were released using Accutase or AccuMax (Innovative Cell Technologies). After staining with trypan blue, viable cells were counted using a hemocytometer.

### Analysis of cTnT-Positive Cells with Flow Cytometer

A cell cluster was released using AccuMax, and then 0.5 to 2 × 10⁶ cells were suspended in a Fixation/Permeabilization solution and allowed to stand (4°C, for 30 minutes or over a night). The cells were washed twice with a Perm/Wash™ buffer. Next, Anti-Troponin, Cardiac Isoform Mouse-Mono (13-11), Ab-1 (1:100), or Purified Mouse IgG1, k Isotype Ctrl was added as a primary antibody, and allowed to stand (4°C, for 30 minutes or over a night). The cells were washed twice with a Perm/Wash™ buffer. Next, Alexa Flour 488 goat anti-mouse IgG1 (1 : 100) was added as a secondary antibody. The cells were washed twice with a Perm/Wash™ buffer. The cells were washed once with 2% FBS in PBS and re-suspended, and the sample was measured and analyzed using Cell Sorter SH800 (Sony).

### Example 1: Influence of Presence of EB Formation on Induction of Differentiation into Cardiomyocytes

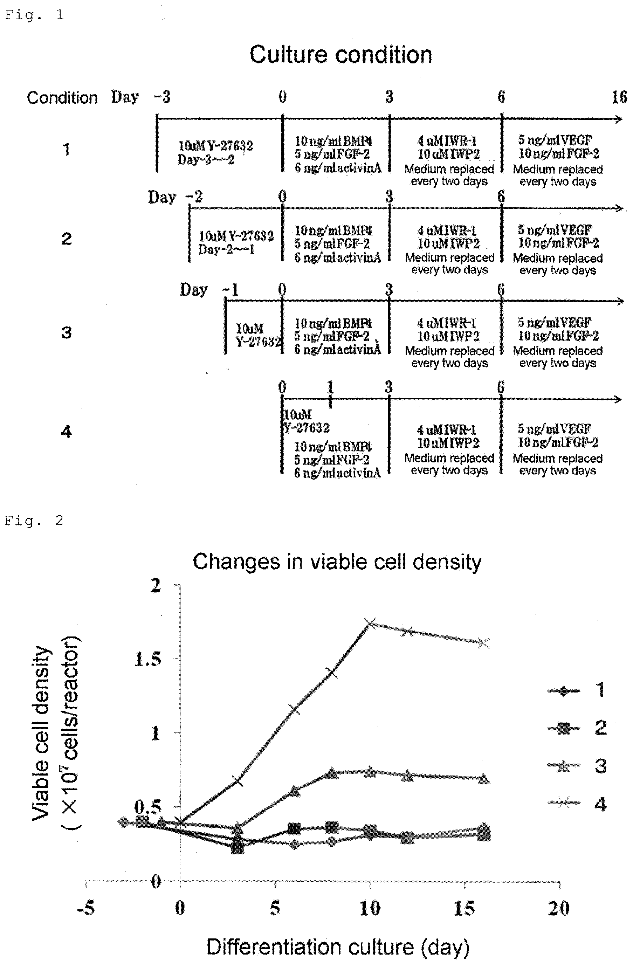

A medium obtained by adding trisodium L-ascorbyl 2-phosphate (final concentration: 50 µg/mL), 2 mM L-glutamine, and 400 µM 1-thioglycerol to StemPro®-34 SFM (Thermo Fisher Scientific) was used (hereinafter referred to as "basal medium"). Under the culture conditions outlined in Fig. 1 (Conditions 1 to 4), iPS cells were induced to differentiate into cardiomyocytes.

Previously cultured iPS cells were released by Accutase treatment, recovered, and then centrifuged to remove the supernatant. The recovered iPS cells were suspended in the medium. In Conditions 1 to 3, iPS cells were suspended in a basal medium comprising 10 µM Y-27632. In Condition 4, iPS cells were suspended in a basal medium comprising 10 µM Y-27632, 10 ng/mL BMP-4, 5 ng/mL FGF-2, and 6 ng/mL activin A. The suspended iPS cells were seeded in a Single use bio-reactor for 30 mL (Biott Corporation) at 4.0 × 10⁶ cells/reactor, and cultured at an agitation rate of 55 rpm and 37°C / 5% CO₂.

In Conditions 1 to 3, culture was performed for 1 day in a basal medium comprising 10 µM Y-27632 (Condition 1: Day -3 to -2, Condition 2: Day -2 to -1, Condition 3: Day -1 to 0). After culturing, in Conditions 1 and 2, culture was further performed for 2 days and 1 day, respectively, in the basal medium (Condition 1: Day -2 to 0, Condition 2: Day -1 to 0). Subsequently, culture was performed for 3 days in a basal medium comprising 10 ng/mL BMP-4, 5 ng/mL FGF-2, and 6 ng/mL activin A (Conditions 1 to 3: Day 0 to 3).

In Condition 4, culture was performed for one day in a basal medium comprising 10 µM Y-27632, 10 ng/mL BMP-4, 5 ng/mL FGF-2, and 6 ng/mL activin A (Condition 4: Day 0 to 1), and then for 2 days in a basal medium comprising 10 ng/mL BMP-4, 5 ng/mL FGF-2, and 6 ng/mL activin A (Condition 4: Day 1 to 3).

After culturing under each condition, cell clusters were recovered from the reactor and transferred to a centrifuging tube. Cell clusters were washed in the basal medium, then resuspended in a basal medium comprising 4 µM IWR-1 and 10 µM IWP-2, and cultured for 3 days (Conditions 1 to 4: Day 3 to 6). Subsequently, the medium was replaced with a basal medium comprising 5 ng/mL VEGF and 10 ng/mL FGF-2, and culture was performed for maximum ten days (Conditions 1 to 4: Day 6 or more). During culturing in these media, the medium was replaced every two days.

The maximum viable cell density under each condition was as shown in the following table (Fig. 2).

**[Table 1]**

| | (× 10⁷ cells/reactor) |
|---|---|
| Condition 1 | 0.361 |
| Condition 2 | 0.341 |
| Condition 3 | 0.741 |
| Condition 4 | 1.74 |

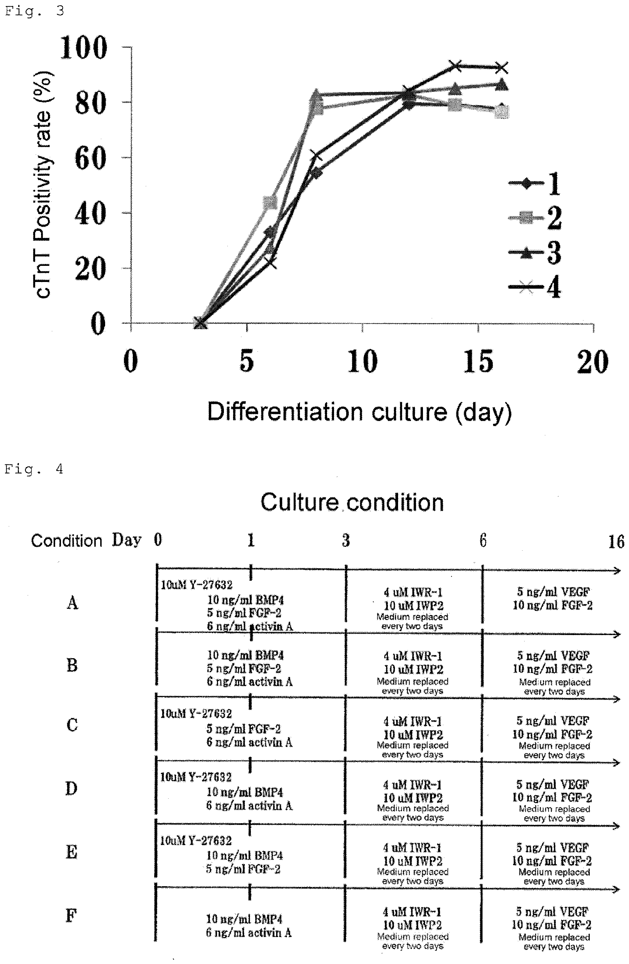

In addition, the maximum cardiomyocyte marker (Cardiac Troponin T: cTnT) was as shown in the following table (Fig. 3) .

**[Table 2]**

| | (%) |
|---|---|
| Condition 1 | 79.56 |
| Condition 2 | 82.82 |
| Condition 3 | 86.75 |
| Condition 4 | 93.38 |

From the above results, it was shown that as compared with Conditions 1 to 3 having the EB formation period, under Condition 4 having no EB formation period, the number of viable cells was larger, and the number of cTnT-positive cells was also larger.

### Example 2: Influence of Difference in Factor Added to Basal Medium on Differentiation Induction Efficiency

The factors shown in the following table were added to the basal medium.

**[Table 3]**

| | Factors added | Final concentration |
|---|---|---|
| Condition A | Y-27632 | 10 µM |
| | BMP-4 | 10 ng/mL |
| | FGF-2 | 5 ng/mL |
| | Activin A | 6 ng/mL |
| Condition B | BMP-4 | 10 ng/mL |
| | FGF-2 | 5 ng/mL |
| | Activin A | 6 ng/mL |
| Condition C | Y-27632 | 10 µM |
| | FGF-2 | 5 ng/mL |
| | Activin A | 6 ng/mL |
| Condition D | Y-27632 | 10 µM |
| | BMP-4 | 10 ng/mL |
| | Activin A | 6 ng/mL |
| Condition E | Y-27632 | 10 µM |
| | BMP-4 | 10 ng/mL |
| | FGF-2 | 5 ng/mL |
| Condition F | BMP-4 | 10 ng/mL |
| | Activin A | 6 ng/mL |

Previously cultured iPS cells were released by Accutase treatment, recovered, and then centrifuged to remove the supernatant. The recovered iPS cells were suspended in each of the media of Conditions A to F, then seeded in a Single use bio-reactor for 30 mL at 5.07 × 10⁶ cells/reactor, and cultured at an agitation rate of 55 rpm and 37°C / 5% CO₂. The culturing procedures under each condition are outlined in Fig. 4.

In Conditions A and C to E, culture was performed for one day in the medium of each condition (Conditions A and C to E: Day 0 to 1), then the medium was replaced with a medium excluding 10 µM Y-27632, and culture was performed for 2 days (Conditions A and C to E: Day 1 to 3). In Conditions B and F, culture was performed for one day under each condition (Conditions B and F: Day 0 to 1), then the medium was exchanged, and culture was further performed for 2 days under the same condition (Conditions B and F: Day 1 to 3).

After culturing under each condition, cell clusters were recovered from the reactor and transferred to a centrifuging tube. Cell clusters were washed in the basal medium, then resuspended in a basal medium comprising 4 µM IWR-1 and 10 µM IWP-2, and cultured for 3 days (Conditions A to F: Day 3 to 6). Subsequently, the medium was replaced with a basal medium comprising 5 ng/mL VEGF and 10 ng/mL FGF-2, and culture was performed for maximum 10 days (Conditions A to F: Day 6 or more).

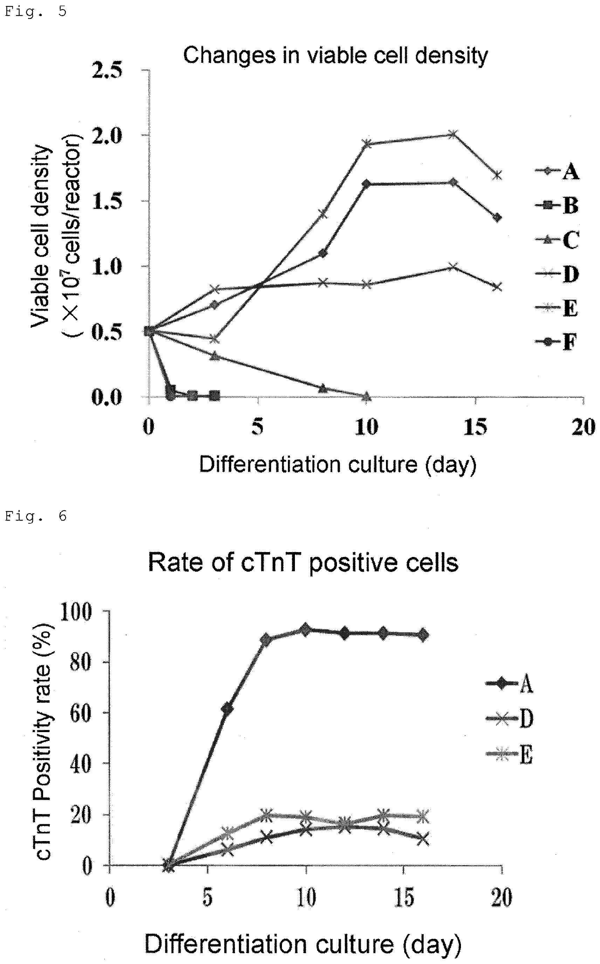

The maximum viable cell density under each condition was as shown in the following table (Fig. 5).

**[Table 4]**

| | (× 10⁷ cells/reactor) |
|---|---|
| Condition A | 1.641 |
| Condition B | -* |
| Condition C | -* |
| Condition D | 0.998 |
| Condition E | 2.007 |
| Condition F | -* |

| | |
|---|---|
| *: Cells died during differentiation induction. | |

In addition, the maximum cardiomyocyte marker (Cardiac Troponin T: cTnT) under each condition where the cells survived was as shown in the following table (Fig. 6).

**[Table 5]**

| | (%) |
|---|---|
| Condition A | 92.84 |
| Condition D | 15.45 |
| Condition E | 19.84 |

### Example 3: Measurement of Cell Beating

A culture solution (2 ml) comprising cardiomyocyte clusters obtained by differentiation induction under the conditions of Condition A of Example 2 was transferred to a 12-well plate MICROPLATE with Lid (IWAKI), and observed under Leica DMi1 inverted microscope (Leica). As a result, autonomous beating of each cardiomyocyte cluster at constant periods was confirmed.

### Example 4: Measurement of Ca⁺ Imaging

Using Calcium Kit-Fluo 4 (Dojindo Laboratories), cell clusters and cells attached onto a substrate were subjected to Ca⁺ imaging. Under the conditions of Condition A of Example 2, differentiation was induced for 16 days to obtain cell clusters. The obtained cell clusters were washed with PBS, then suspended in Loading Buffer, and transferred to MATUNAMI GLASS BOTTOM DISH Hydro 35-mm dish (Matsunami Glass Ind., Ltd.). Subsequently, incubation was performed at 37°C for 1 hour. After incubation, Loading Buffer was removed, followed by washing with PBS. The obtained cell clusters were transferred to Recording Medium and subjected to fluorescent observation under ECLIPSE Ts2 (Nikon). Similarly, cell clusters obtained by differentiation induction for 16 days under the conditions of Condition A of Example 2 were treated with AccuMax (Innovation cell technologies) to release the cell clusters. The released cells clusters were suspended in DMEM+10% FBS, and the suspension was passed through a 40-µm strainer (FALCON). On MATUNAMI GLASS BOTTOM DISH Hydro 35-mm dish previously coated with human recombinant laminin-221 (0.5 µg/cm², VERITAS), cells diluted with DMEM + 10% FBS were seeded to make 1 × 10⁶ cells/well and cultured for 7 days in DMEM + 10% FBS. Subsequently, by the same operation as above, cells attached onto a substrate were subjected to Ca⁺ imaging. As a result, in the cell clusters and the attached cells, an increase in the Ca⁺ concentration in synchronization with beating was confirmed.

### Example 5: Expression of Myocardial Marker

Cell clusters obtained by differentiation induction for 16 days under the conditions of Condition A of Example 2 were treated with AccuMax (Innovation cell technologies) to release the cell clusters. The cell clusters were suspended in DMEM + 10% FBS, and the suspension was passed through a 40-µm strainer (FALCON). On a 24-well plate MICROPLATE with Lid (IWAKI) previously coated with fibronectin (5 µg/cm², Sigma), cells diluted with DMEM + 10% FBS were seeded to make 4 × 10⁴ cells/well and cultured for 6 days in DMEM + 10% FBS. The culture supernatant was removed, and, after washing with PBS three times, the cells were fixed with a Fixation/Permeabilization solution (4°C, 60 minutes or one night). After washing with PBS three times, blocking with PBS + 3% FBS was performed for 30 minutes at room temperature. The liquid was discarded and replaced with PBS + 1% FBS comprising various antibodies (4°C, 60 minutes or one night). The liquid was discarded, replaced with 0.1% FBS/PBST, and allowed to stand for 5 minutes, followed by washing (static washing). This washing operation was performed three times. After removing the washing liquid, various secondary antibodies were added (room temperature, for 60 minutes). After 5-minute static washing with PBS was performed three times, cells were treated with DAPI (Life technologies) (room temperature, for 15 minutes). After washing with PBS three times, fluorescence measurement was performed with EVOS FL Auto (Life technologies). The used antibodies and their concentrations are shown below.

### [Antibodies used and Concentrations thereof]

Anti-Troponin Cardiac Isoform Mouse-Mono (13-11), Ab-1 (Thermo Fisher Scientific): 2 µg/ml (diluted 100-fold)
Anti-Sarcomeric Alpha Actinin ab9465 (abacam): 2.5 µg/ml (diluted 50-fold)
Purified Mouse IgG1, k Isotype Control (Biolegend): 2 µg/ml or 2.5 µg/ml
Myosin Heavy Chain (MHC) Antibody (R&D systems): 2.5 µg/ml (diluted 200-fold)
Negative Control Mouse IgG2b (Dako): 2.5 µg/ml
Alexa Fluor 488 A21121 and Alexa Fluor 488 A11001 (Thermo Fisher Scientific): 20 µg/ml (diluted 100-fold)

As a result, the expression of cTnT (myocardial marker), α-Actinin, and MHC was confirmed in the differentiation-induced iPS-derived cardiomyocytes.

### Example 6: Measurement of Extracellular Potential

Cell clusters obtained by differentiation induction for 14 days under the conditions of Condition A of Example 2 were treated with AccuMax (Innovation cell technologies) to release the cell clusters. The cell clusters were suspended in DMEM + 10% FBS, and the suspension was passed through a 40-µm strainer (FALCON). A frame was previously prepared from silicon to enclose the electrode of multi-electrode dish MED-P 530A (Alpha MED Scientific Inc.), and then the inside was coated with fibronectin (about 5 µg/cm², Sigma). 1 × 10⁵ cells were seeded in the frame and allowed to stand for 1 hour in a 5% CO₂ incubator at 37°C. After confirming the sedimentation and attachment of cells, the medium was added, and culture was performed for 4 days. The extracellular potential was measured with MED64 (Alpha MED Scientific Inc.). As a result, the occurrence of autonomous depolarization and repolarization in the induced cardiomyocytes was confirmed.

From the above results, it was shown that when iPS cells are suspension-cultured using a medium comprising the above four factors (ROCK inhibitor, BMP4, FGF-2, and activin A), mesodermal cells can be induced without through preparation step such as EB formation, and the obtained mesodermal cells can be induced into cardiomyocytes. In addition, it was also shown that cardiomyocytes can be induced at a high survival ratio with high efficiency. Further, autonomous beating of the induced cardiomyocytes at constant periods was confirmed. Further, the occurrence of autonomous depolarization and repolarization in the induced cardiomyocytes was also confirmed. That is, it was shown that by using the method of the present invention, cardiomyocytes can be induced with high efficiency in a quick and simple manner. In the case of using suspension culture, this method is also suitable for the mass culture of cells.

### Example 7: Differentiated Stem Cells of Cardiomyocytes and Culture Method using iPS Cells Cultured under Feeder-Free Conditions

### Stem Cells and Culture Method

As stem cells, an iPS cell strain 253G1 (pluripotent stem cells) was used (Nakagawa, M. et al., Nature Biotechnology 26: 101-106 (2008)). The feeder-free culture of iPS cells was performed under the following conditions. The incubator was coated with iMatrix® 511 at 0.5 ng/cm². As the medium for iPS cells, StemFit® AK02N was used. Cells were released with 0.5 × TrypLE™, and then subcultured in a medium obtained by adding 10 µM of Y-276314 to StemFit® AK02N at 4-8 × 10⁴ cells/T-25 flask (Corning). After the first day of culture, the medium was replaced with StemFit® AK02N to maintain the culture.

The cultured cells were washed with PBS, and then the cells were released with 0.5 × TrypLE™. Subsequently, the supernatant was removed by centrifugation. The recovered iPS cells were suspended in the medium of Condition A of Example 2, then seeded in a Single use bio-reactor for 30 mL at 5.0 × 10⁶ cells/reactor, and cultured at an agitation rate of 55 rpm and 37°C / 5% CO₂. Culture was performed for 3 days in the medium of the Condition A of Example 2. Subsequently, cell clusters were recovered from the reactor and transferred to a centrifuging tube. The supernatant was removed, and cell clusters were washed once in the basal medium, then resuspended in a basal medium comprising 4 µM IWR-1 and 10 µM IWP-2, and cultured again in the reactor for 3 days. After culturing, the medium was replaced with a basal medium comprising 5 ng/mL VEGF and 10 ng/mL FGF-2, and culture was performed for maximum 15 days. During culturing, the medium was replaced every two days.

The cardiomyocyte marker (Cardiac Troponin T: cTnT) in each differentiation induction period was as shown in the following table.

**[Table 6]**

| | | | | |
|---|---|---|---|---|
| Induction period (day) | 9 | 12 | 15 | 21 |
| cTnT Positivity rate (%) | 91.98 | 94.04 | 93.65 | 95.82 |

From the above results, it was shown that also from iPS cells cultured under feeder-free conditions using the medium and method of the present invention, mesodermal cells can be induced without through EB formation or like preparation steps, and the obtained mesodermal cells can be induced into cardiomyocytes with high efficiency.

### Industrial Applicability

According to the present invention, stem cells can be induced to differentiate into mesodermal cells, such as cardiomyocytes, with high efficiency in a quick and simple manner. In the case of using suspension culture, this method is also suitable for the mass culture of mesodermal cells. Therefore, the present invention is useful in the field of regenerative medicine. In addition, the present invention is also useful in the development of drugs.

## Claims

1. A medium for inducing the differentiation of stem cells into mesodermal cells, comprising a ROCK inhibitor, a bone morphogenetic protein (BMP), a fibroblast growth factor (FGF), and an activin.

2. The medium according to claim 1, wherein the ROCK inhibitor is Y-27632.

3. The medium according to claim 1 or 2, wherein the BMP is BMP4.

4. The medium according to any one of claims 1 to 3, wherein the FGF is FGF-2.

5. The medium according to any one of claims 1 to 4, wherein the activin is activin A.

6. The medium according to any one of claims 1 to 5, wherein the mesodermal cells are cardiac progenitor cells or cardiomyocytes.

7. The medium according to any one of claims 1 to 6, wherein the stem cells are pluripotent stem cells or mesenchymal stem cells.

8. A method for producing mesodermal cells from stem cells, comprising a step of culturing stem cells in the medium according to any one of claims 1 to 7.

9. A method for producing cardiac progenitor cells or cardiomyocytes from stem cells, comprising:
a step (1) of culturing stem cells in the medium according to any one of claims 1 to 7, and then;
a step (2) of culturing in a medium comprising a Wnt inhibitor.

10. The method according to claim 9, wherein the Wnt inhibitor is IWR-1 and IWP-2.

11. The method according to any one of claims 8 to 10, wherein the culture is suspension culture.

12. The method according to any one of claims 8 to 11, wherein the stem cells are pluripotent stem cells or mesenchymal stem cells.

13. A composition for assisting the induction of the differentiation of stem cells into mesodermal cells, comprising a ROCK inhibitor, a BMP, an FGF, and an activin.

14. A cell group obtainable by the method according to any one of claims 8 to 12, comprising 90% or more mesodermal cells.
